# DEMANDE DE BREVET EUROPEEN

(11) **EP 1 736 176 A1**
(43) Date de publication de la demande: **27.12.2006**
(21) Numéro de dépôt: 05447144.6
(22) Date de dépôt: 21.06.2005
(51) Int. Cl.: A61L 2/20, C01B 13/11, C01B 13/10, A61L 9/015

(54) **Appareil générateur d'ozone dans un gaz porteur inerte**

(71) Demandeur: Druart, Philippe, 4020 Liege (BE)
(72) Inventeur: Druart, Philippe, 4020 Liege (BE)
(74) Mandataire: Van Malderen, Michel

(57) **Abrégé**

La présente invention se rapporte à un appareil générateur d'ozone pour la stérilisation ou la désinfection dans lequel l'ozone est généré sous forme gazeuse par décharge corona dans un générateur haute tension à partir d'air, caractérisé en ce qu'il comprend des moyens pour réchauffer et propulser un gaz inerte initialement sous forme liquide et le mélanger à l'ozone produit, de sorte que le gaz inerte constitue un gaz porteur de l'ozone sortant de l'appareil sous forme d'un mélange à une pression totale comprise entre 2 et 10 bar et à une température inférieure d'au moins 10°C à la température ambiante.

## Description

### Objet de l'invention

La présente invention se rapporte à un appareil capable de stériliser les instruments chirurgicaux de toute nature lorsque leurs composants résistent à une oxydation prolongée. Son utilisation vise tout spécialement les instruments "réutilisables" de la famille des endoscopes et autres, après intervention chirurgicale et/ou simplement diagnostique. En particulier, l'invention peut être utilisée lors d'interventions chirurgicales humaines ou animales, directement sur le champ opératoire "ouvert".

L'invention relève également d'autres domaines comme celui de la désinfection des bâtiments, particulièrement les hôpitaux (salles d'opération, chambres de malade, couloirs, etc.) mais aussi les usines agroalimentaires et les abattoirs, leurs instruments, machines et produits ainsi que celui de la stérilisation des cultures maraîchères, fruitières et agricoles en général.

### Etat de la technique

L'effet oxydant, bactéricide, fongicide, antiviral, désinfectant, stérilisant, etc., de l'ozone (oxygène triatomique) est bien connu.

L'ozonation est un traitement chimique par oxydation. A côté d'autres réactifs chimiques oxydants utilisés pour la destruction des germes pathogènes, l'ozone a l'avantage de présenter une action complémentaire dans la destruction d'un grand nombre de micropolluants, dans l'amélioration des goûts, des odeurs et dans la destruction des colorants.

L'instabilité de l'ozone impose sa production sur le lieu de consommation. A l'échelle industrielle, l'ozone est produit dans un générateur à tube à décharge électrique (haute tension), dans lequel on fait circuler de l'air ou de l'oxygène sec.

Les installations actuelles de production d'ozone sont basées sur la bonne solubilité de l'ozone dans l'eau (voir par exemple document EP-A-1 491 495). L'eau est donc généralement utilisée comme vecteur pour l'ozone à des fins de traitement thérapeutique, phytosanitaire, désinfectant, etc.

Toutefois, une difficulté technique essentielle consiste alors à trouver la dose optimale d'ozone dissous pour éviter l'obtention d'une solution soit trop oxydante, soit inefficace lors du traitement. Par exemple, il importe de maîtriser la haute tension d'alimentation des tubes générateurs, la température et la pression de l'alimentation en eau courante. De plus, pratiquement, des problèmes de sécurité surgissent inévitablement du fait d'une certaine proximité de l'eau et de la haute tension.

L'ozone étant une molécule chimique instable, sa demi-vie dans l'eau à température ambiante est de l'ordre de 15-20 minutes. Il est connu que la demi-vie et la solubilité de l'ozone dans l'eau diminuent avec la température. Ainsi, il n'est plus possible d'utiliser l'ozone dissous dans l'eau au-delà de 40°C. Dans l'air, il est difficile pratiquement d'obtenir une demi-vie supérieure à 60 minutes, sauf dans un environnement stérile et à basse température.

Par ailleurs, la problématique de plus en plus importante des infections nosocomiales, c'est-à-dire contractées en milieu hospitalier, incite à trouver de nouvelles solutions plus efficaces, moins toxiques et/ou plus rapides tant pour la désinfection des locaux et du matériel hospitalier, que pour la stérilisation des instruments médicaux et chirurgicaux.

Ainsi, dans le domaine de la stérilisation, la stérilisation à la vapeur sous pression (autoclave) ou à l'air chaud est longue (par exemple respectivement 20 minutes à 121°C et 60 minutes à 200°C) et ne s'applique qu'au matériel supportant des températures et pressions élevées, et le cas échéant, l'humidité ; la stérilisation au gaz (oxyde d'éthylène, vapeur de formaldéhyde) ou par immersion (glutaraldéhyde, acide peracétique) est souvent toxique ou présente un danger d'explosion; d'autres techniques encore peuvent s'avérer très coûteuses (stérilisation au peroxyde d'hydrogène, par flux de plasma froid) et/ou soumises à des normes de sécurité très sévères (radiostérilisation).

La société TSO₃ Inc. (Sainte-Foy, Québec, Canada ; www.tso3.com) a conçu un appareil de stérilisation adapté à la nouvelle génération d'instruments chirurgicaux et diagnostiques composés de matériaux sensibles à la chaleur, en particulier les polymères. Cet appareil fonctionne à une température sensiblement supérieure à la température ambiante (> 30°C). Une chambre de stérilisation est remplie d'instruments et éventuellement d'emballages compatibles. Le cycle de stérilisation commence par un passage sous vide suivi d'une phase d'humidification de la chambre, au moyen de vapeur d'eau générée par un humidificateur. Ensuite, l'ozone fabriqué dans un générateur à décharge corona à partir d'oxygène de qualité médicale, comprimé et séché, est injecté à l'intérieur de la chambre et la stérilisation commence. A la moitié du cycle, les phases allant de l'étape du vide à l'injection d'ozone se répètent et sont suivies par une phase de ventilation pour que l'ozone soit évacué de la chambre et des emballages. Au cours du cycle de stérilisation, l'ozone est transformé en oxygène grâce à un catalyseur. Seuls de l'oxygène et de la vapeur d'eau purifiée sont rejetés à l'atmosphère. Le principal inconvénient de cet appareil est la durée du cycle de stérilisation qui est d'environ 4 heures et 15 minutes à une température de 30-35°C.

### Buts de l'invention

La présente invention vise à fournir une solution qui permette de s'affranchir des inconvénients de l'état de la technique.

En particulier, l'invention a pour but de fournir des moyens de stérilisation et désinfection permettant de s'attaquer efficacement aux causes des maladies nosocomiales.

Un but complémentaire de l'invention est de fournir un générateur où l'ozone est produit dans des conditions permettant une désinfection ou une stérilisation plus rapides et plus efficaces par comparaison à l'ozone dissous dans l'eau ou à d'autres méthodes.

### Principaux éléments caractéristiques de l'invention

La présente invention se rapporte à un appareil générateur d'ozone pour la stérilisation ou la désinfection dans lequel l'ozone est généré sous forme gazeuse par décharge corona dans un générateur haute tension à partir d'air, caractérisé en ce qu'il comprend des moyens pour réchauffer et propulser un gaz inerte initialement sous forme liquide et le mélanger à l'ozone produit, de sorte que le gaz inerte constitue un gaz porteur de l'ozone sortant de l'appareil sous forme d'un mélange à une pression totale comprise entre 2 et 10 bar et à une température inférieure d'au moins 10°C à la température ambiante, de préférence à une température comprise entre -30°C et +10°C.

Selon une première modalité d'exécution préférée de l'invention, l'appareil comprend un premier dispositif se composant au moins d'une chambre contenant le gaz inerte, de préférence de l'azote, sous forme liquide, d'un réchauffeur intégré à une interparoi, d'une chambre pressurisée contenant le gaz inerte vaporisé par réchauffement et d'un régulateur de pression qui fournit le gaz inerte à une sortie sous une pression inférieure à 8 bars et à une température inférieure à -100°C.

Avantageusement, l'appareil de l'invention comprend un second dispositif se composant au moins d'une entrée d'air ambiant, un compresseur pour comprimer l'air entrant dans un conduit en communication avec un générateur d'ozone alimenté par une source de haute tension, piloté par un régulateur électronique et qui fournit de l'ozone à une sortie.

De préférence, cet appareil comprend un conduit ou une chambre de mélange de l'ozone produit et du gaz inerte sous pression.

Toujours avantageusement, le second dispositif est configuré pour fournir jusqu'à 2000 mg O³/heure à un débit de 10 litres/min.

Selon une modalité d'application très avantageuse, cet appareil comprend en outre une chambre réfrigérée à une température inférieure ou égale à 4°C, dans laquelle est injecté par au moins une entrée le mélange d'ozone et de gaz inerte précité.

De préférence, la chambre réfrigérée comprend des grilles et des fils de suspension permettant de stériliser des instruments médicaux ou chirurgicaux, se trouvant dans la chambre, éventuellement avec des articles d'emballage tels que des sachets dans lesquels ils seront emballés de manière stérile.

De préférence encore, la chambre réfrigérée comprend une roulette obturant une entrée du mélange de gaz précité, munie d'orifices et configurée pour permettre le passage du gaz à l'intérieur de tubes ou tuyaux accrochés à la roulette.

Toujours avantageusement, la chambre réfrigérée comprend une porte d'ouverture avant et une porte en verre se trouvant derrière ladite porte avant et munie d'orifices à manches pour la manipulation stérile d'objets se trouvant à l'intérieur de la chambre réfrigérée, de préférence pour l'emballage stérile desdits objets.

Toujours selon l'invention, la chambre réfrigérée comprend une vanne munie d'un clapet anti-retour pour l'évacuation, avant ouverture de la porte avant, du gaz contenu dans la chambre réfrigérée vers un réducteur-destructeur d'ozone avant sortie vers l'ambiance.

Selon une seconde modalité d'exécution préférée de l'invention, l'appareil comprend un tuyau de préférence flexible connecté au conduit ou à la chambre de mélange de l'ozone produit et du gaz inerte sous pression, pour diffuser ledit gaz vers l'extérieur.

L'invention concerne également une installation comprenant l'appareil selon la seconde modalité d'exécution, caractérisée en ce qu'elle comprend un système de dispersion par faisceau connecté au conduit ou à la chambre de mélange de l'ozone produit et du gaz inerte sous pression, ledit système étant situé au-dessus et au-dessous d'un convoyeur à bande sous aération et transportant des objets à stériliser ou désinfecter.

Un objet complémentaire de l'invention se rapporte à une utilisation d'un appareil selon la première modalité d'exécution préférée, pour la stérilisation de matériel médical, chirurgical ou dentaire.

Un autre objet encore complémentaire de l'invention se rapporte à une utilisation de l'appareil selon la seconde modalité d'exécution préférée, pour la désinfection de locaux et de matériel, de préférence dans le secteur hospitalier et agroalimentaire.

Un autre objet encore complémentaire de l'invention se rapporte à une utilisation de l'appareil selon la seconde modalité d'exécution préférée, lors d'interventions chirurgicales humaines ou animales, directement sur le champ opératoire "ouvert".

Un autre objet encore complémentaire de l'invention se rapporte à une utilisation de l'installation précitée pour la stérilisation de produits agroalimentaires.

### Brève description des figures

La figure 1 représente schématiquement une première forme d'exécution préférée de l'appareil selon la présente invention.

La figure 2 montrent les colonies restantes de souche A *(Staphylococcus aureus* ATCC 43300) sur boîte de pétri à différentes durées de désinfection au moyen de l'appareil selon la présente invention (variante 2).

### Description de formes d'exécution préférées de l'invention

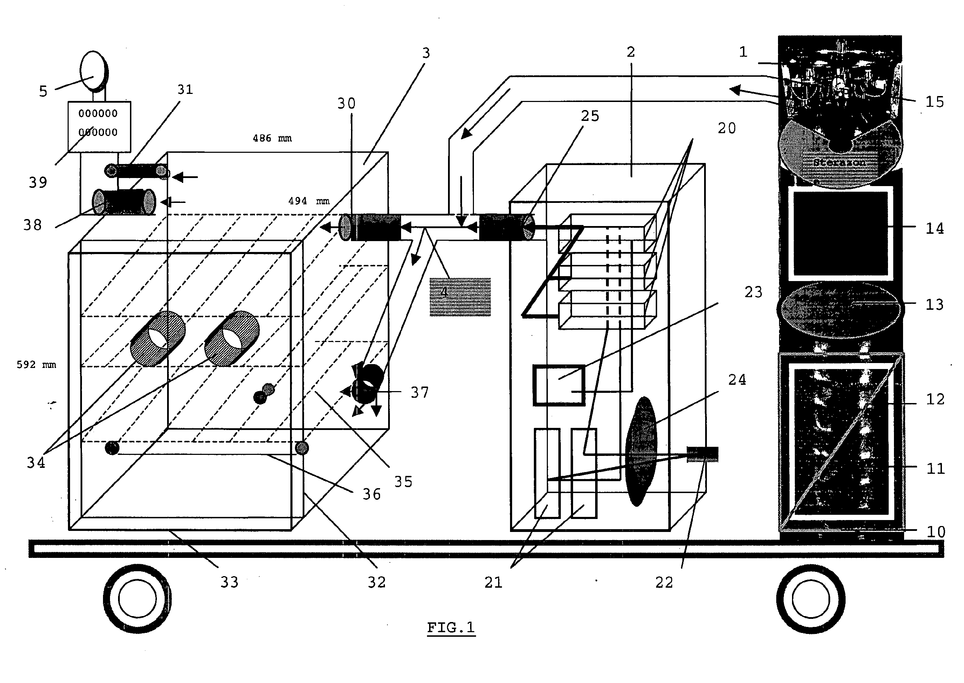

Selon une première forme d'exécution préférée de l'invention adaptée à la stérilisation et représentée sur la figure 1, l'appareil de l'invention est lui-même un assemblage de deux dispositifs distincts :
- un premier dispositif 1 est composé d'un récipient en acier inoxydable 10 présentant une chambre 11 contenant de l'azote liquide. Un réchauffeur 12 intégré à l'interparoi 13 chauffe l'azote liquide à la base du récipient, ce qui le gazéifie et le fait monter dans une chambre pressurisée supérieure 14 se trouvant à une température de -172°C. Le gaz alors prêt à l'emploi est extrait du récipient 14 à une pression de 4 bars (max. 6-8 bars) contrôlée par un régulateur de pression 15.
   L'azote, gaz inerte très peu réactif, est utilisé comme propulseur de l'ozone, gaz très réactif et volatil, créé par un second dispositif et auquel il est mélangé. L'azote va donc servir de vecteur à l'ozone (pression partielle = 1,5 bar) ;
- un second dispositif 2 comprend un triple générateur électronique d'ozone qui produit par une sortie 25, dans le cas de la stérilisation, 400 à 800 mg O³/h à un débit de 10 litres/min. Il est composé de trois générateurs 20 alimentés par une source de haute tension 24, de deux minicompresseurs 21 qui aspirent l'air ambiant à une entrée 22, ceux-ci étant gérés par une carte électronique de régulation 23. Selon la figure 1, ce dispositif est directement jumelé à un conteneur réfrigéré du type petit frigo 3 (par exemple de contenance 28 litres, dimensions H x L x P = 580 mm x 422 mm x 393 mm et poids 16kg ou de contenance 53 litres, dimensions H x L x P = 592 mm x 486 mm x 494 mm et poids 18kg suivant la nécessité), dans lequel pénètre par une entrée 30 le mélange 4 des deux gaz précités. Ce conteneur présente une soupape de sécurité 31 qui permet au gaz injecté 4 de s'échapper en cas de surpression. Une porte en verre 32 se trouve derrière la porte d'ouverture avant 33. Elle contient deux orifices avec manches 34 pour permettre les manipulations nécessaires à l'intérieur du conteneur avant ouverture de la porte avant. De cette façon, il est possible de placer les instruments chirurgicaux stérilisés dans des pochettes totalement stériles se trouvant déjà à l'intérieur du conteneur.

Les instruments à stériliser sont avantageusement soit déposés sur des grilles 35, soit suspendus à des fils 36 dans le conteneur 3, pour permettre au gaz d'agir sur toutes leurs surfaces. Les tuyaux, par exemple d'endoscopes, sont quant à eux attachés à une roulette 37 présentant des orifices de différents diamètres, directement reliés à l'arrivée du gaz 4. Ceci permet une stérilisation à l'intérieur même des tubes ou tuyaux.

Les sorties de gaz des deux dispositifs décrits ci-dessus sont assemblées pour conduire les deux gaz, azote et ozone, dans un seul conduit à l'intérieur du conteneur réfrigéré. L'azote sortant à environ -120°C refroidit instantanément l'ozone, lequel est produit directement à une température inférieure d'environ 12°C par rapport à la température ambiante. Comme ce "nouveau" gaz entre dans un air à environ 2°C (conteneur réfrigéré), la capacité de stérilisation de l'ozone est ainsi nettement améliorée et son action sera d'autant plus efficace.

Ce traitement est particulièrement efficace pour les instruments ne résistant pas à des températures élevées. Il permet donc une réutilisation plus longue de ces instruments et donc un meilleur amortissement des investissements en matériel.

Le conteneur comprend encore une vanne d'évacuation du gaz qu'il contient, avec clapet anti-retour 38, vers un destructeur-réducteur d'ozone 39 relié par une sortie 5 à l'ambiance, cette vanne devant être ouverte avant l'ouverture de la porte avant. Ceci permet d'éviter toute odeur ou tout niveau d'ozone trop élevé dans la pièce où se trouve l'appareil de l'invention.

Selon une seconde forme d'exécution préférée de l'invention, adaptée à la désinfection des hôpitaux (salles d'opération, chambres de malades, couloirs, etc.), des usines et machineries de l'agroalimentaire, etc., l'appareil de l'invention ne nécessite pas de conteneur de type frigo.

Le dispositif permettant la vaporisation de l'azote liquide est par ailleurs le même que celui utilisé pour la stérilisation. L'azote est propulsé à une pression pouvant aller jusqu'à 8 bars. Il est ensuite mélangé à l'ozone dès la production de celui-ci qui est réalisée en quantité doublée par rapport à celle produite dans l'appareil de stérilisation (800 à 1600 mg O³/h à 10 litres/min.). Le mélange des deux gaz, sortant d'un diffuseur comprenant un tuyau de préférence flexible, asperge le volume à désinfecter selon un jet dispersé contre les murs, plafonds et sols ou autres cibles pour éliminer rapidement un maximum de microorganismes pathogènes. L'opérateur est protégé par un masque. L'opération une fois terminée, il suffit d'ouvrir une fenêtre pour faciliter la désintégration de l'ozone et sa transformation en oxygène, ainsi que la ventilation, par apport d'air frais.

Dans l'agroalimentaire, on prévoira un système de dispersion par faisceaux sur les aliments à traiter, se trouvant au-dessus et au-dessous d'un tapis roulant très aéré, afin d'éliminer toute forme de microorganismes nocifs (bactéries, virus, champignons, etc.). La technique selon l'invention prolonge la vie des aliments en tuant toute bactérie qui ne peut donc se développer. Dans ce cas, il faudra prévoir que le générateur d'ozone produise une quantité moyenne d'ozone comprise typiquement entre 600 et 1000 mg O³/h à 10 litres/min.

### ESSAIS SUR BACTERIES MISES EN CULTURE

Afin de tester les deux variantes de l'appareil selon l'invention, sept souches bactériennes ont été mises en culture (Tableau 1).

**Tableau 1**

| ***Souche*** | ***Dénomination*** |
|---|---|
| A | *Staphylococcus aureus* ATCC 43300 |
| 13 | *Staphylococcus aureus* 13136 p⁻m⁺ |
| E | *Enterococcus faecium* EFM-1 |
| H | *Enterococcus faecium* H80721 |
| D | *Enterococcus faecium* D366 |
| B2 | *Bacillus megaterium* |
| B3 | *Bacillus cereus* |

### Matériels et méthodes

Les cultures en erlenmeyer et sur boîte de pétri ont été réalisées en milieu standard BH. Un dosage spectrophotométrique des cultures bactériennes a été effectué par mesure de l'absorbance à 600 nm, la dilution étant réalisée pour obtenir une densité optique < 1. les dilutions ont été effectuées en visant l'obtention de 200 colonies par boîte (1 unité d'absorbance = 10⁹ bactéries). Les cultures de bactéries ont d'abord été incubées à 37°C sous agitation pour A, B2, B3 et 13 et sans agitation pour E, H et D. Elles ont été ensuite diluées et étalées sur des boîtes de pétri avec une incubation généralement à 37°C (28°C pour B2 et B3 qui forment de très grosses colonies).

### Résultats

Un exemple de comptage final des étalements avant traitement des boîtes par stérilisation ou désinfection est donné par le tableau 2 (duplicate).

**Tableau 2**

| ***Souche*** | ***Nombre* de *colonies*** |
|---|---|
| A | 154 |
| A | 139 |
| B2 | 6 |
| B2 | 7 |
| B3 | 0 |
| B3 | 1 |
| D | 168 |
| D | 187 |
| E | 55 |
| E | 55 |
| H | 158 |
| H | 159 |
| 13 | 401 |
| 13 | Sans objet |

Le traitement des boîtes de pétri ouvertes a été réalisé soit sous forme de stérilisation dans une enceinte fermée pendant un temps déterminé (variante 1 de l'invention), soit sous forme de passage au diffuseur gazeux (variante 2 de l'invention). Les résultats sont présentés ci-après dans le tableau 3 (stérilisation) et dans le tableau 4 (désinfection) respectivement.

**Tableau 3**

| ***Souche*** | ***2 min*** | ***4 min*** | ***6 min*** |
|---|---|---|---|
| A | 0 | 0 | 0 |
| B2 | 1 | 1 | 0 |
| B3 | - | 0 | 0 |
| D | 15 | 1 | 0 |
| E | 0 | 0 | 0 |
| H | 0 | 0 | 1 |
| 13 | 197 | 0 | 0 |

**Tableau 4**

| ***Souche*** | ***1 min*** | ***2 min*** | ***3 min*** | ***4 min*** | ***5 min*** |
|---|---|---|---|---|---|
| A | 5 | 24 | 3 | 1 | 7 |
| B2 | 1 | 1 | 0 | 0 | 0 |
| B3 | 0 | 0 | - | 0 | 0 |
| D | 7 | 32 | 0 | 0 | 2 |
| E | 85 | 3 | 1 | 7 | 4 |
| H | 152 | 100 | 0 | 0 | 7 |
| 13 | 14 | 8 | 2 | 46 | 33 |

Certains résultats apparemment aberrants dans le tableau 4 sont probablement liés au fait que les colonies se sont multipliées dans les tubes de mesure. En effet, tous les contrôles n'ont pu être réalisés en même temps et les colonies n'ont pas été conservées sur glace pendant le temps de contrôle.

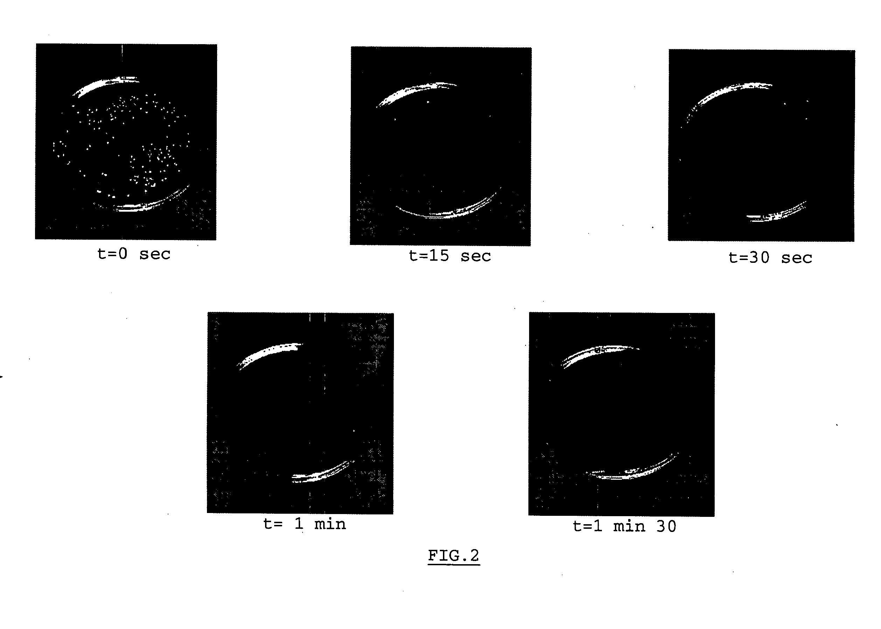

Un exemple de cinétique de désinfection pour la souche A est donné dans le tableau 5, le diffuseur d'ozone étant maintenu fixe au-dessus des boîtes ouvertes après étalement, sauf au temps t=0. Un résultat similaire est présenté sur la figure 2.

**Tableau 5**

| ***Temps de désinfection*** | ***Nombre de colonies*** |
|---|---|
| 0 | 65 |
| 15 s | 2 |
| 30 s | 4 |
| 1 min | 2 |
| 1 min 30 s | 0 |
| 2 min | 0 |

Ces tests ont été réalisés à l'Institut de Chimie de l'Université de Liège.

## Revendications

1. Appareil générateur d'ozone pour la stérilisation ou la désinfection dans lequel l'ozone est généré sous forme gazeuse par décharge corona dans un générateur haute tension à partir d'air, **caractérisé en ce qu'**il comprend des moyens pour réchauffer et propulser un gaz inerte initialement sous forme liquide et le mélanger à l'ozone produit, de sorte que le gaz inerte constitue un gaz porteur de l'ozone sortant de l'appareil sous forme d'un mélange à une pression totale comprise entre 2 et 10 bar et à une température inférieure d'au moins 10°C à la température ambiante, de préférence à une température comprise entre -30°C et +10°C.

2. Appareil selon la revendication 1,
**caractérisé en ce qu'**il comprend un premier dispositif (1) se composant au moins d'une chambre (11) contenant le gaz inerte, de préférence de l'azote, sous forme liquide, d'un réchauffeur (12) intégré à une interparoi (13), d'une chambre pressurisée (14) contenant le gaz inerte vaporisé par réchauffement et d'un régulateur de pression (15) qui fournit le gaz inerte à une sortie (16) sous une pression inférieure à 8 bars et à une température inférieure à - 100°C.

3. Appareil selon la revendication 1 ou 2, **caractérisé en ce qu'**il comprend un second dispositif (2) se composant au moins d'une entrée d'air ambiant (22), un compresseur (21) pour comprimer l'air entrant dans un conduit en communication avec un générateur d'ozone (20) alimenté par une source de haute tension (24), piloté par un régulateur électronique (23) et qui fournit de l'ozone à une sortie (25).

4. Appareil selon la revendication 2 ou 3, **caractérisé en ce qu'**il comprend un conduit ou une chambre de mélange (4) de l'ozone produit et du gaz inerte sous pression.

5. Appareil selon la revendication 3,
**caractérisé en ce que** le second dispositif (2) est configuré pour fournir jusqu'à 2000 mg O³/heure à un débit de 10 litres/min.

6. Appareil selon l'une quelconque des revendications précédentes, **caractérisé en ce qu'**il comprend en outre une chambre réfrigérée (3) à une température inférieure ou égale à 4°C, dans laquelle est injecté par au moins une entrée (30) le mélange d'ozone et de gaz inerte précité (4).

7. Appareil selon la revendication 6,
**caractérisé en ce que** la chambre réfrigérée (3) comprend des grilles (35) et des fils de suspension (36) permettant de stériliser des instruments médicaux ou chirurgicaux, se trouvant dans la chambre, éventuellement avec des articles d'emballage tels que des sachets dans lesquels ils seront emballés de manière stérile.

8. Appareil selon la revendication 6 ou 7, **caractérisé en ce que** la chambre réfrigérée (3) comprend une roulette (37) obturant une entrée (30) du mélange de gaz précité (4), munie d'orifices et configurée pour permettre le passage du gaz à l'intérieur de tubes ou tuyaux accrochés à la roulette.

9. Appareil selon la revendication 6, 7 ou 8, **caractérisé en ce que** la chambre réfrigérée (3) comprend une porte d'ouverture avant (33) et une porte en verre (32) se trouvant derrière ladite porte avant (33) et munie d'orifices à manches (34) pour la manipulation stérile d'objets se trouvant à l'intérieur de la chambre réfrigérée (3), de préférence pour l'emballage stérile desdits objets.

10. Appareil selon l'une quelconque des revendications 6 à 9, **caractérisé en ce que** la chambre réfrigérée (3) comprend une vanne munie d'un clapet anti-retour (38) pour l'évacuation, avant ouverture de la porte avant (33), du gaz (4) contenu dans la chambre réfrigérée (3) vers un réducteur-destructeur d'ozone (39) avant sortie (5) vers l'ambiance.

11. Appareil selon l'une quelconque des revendications 1 à 5, **caractérisé en ce qu'**il comprend un tuyau de préférence flexible connecté au conduit ou à la chambre de mélange (4) de l'ozone produit et du gaz inerte sous pression, pour diffuser ledit gaz vers l'extérieur.

12. Installation comprenant un appareil selon la revendication 11, **caractérisée en ce qu'**elle comprend un système de dispersion par faisceau connecté au conduit ou à la chambre de mélange (4) de l'ozone produit et du gaz inerte sous pression, ledit système étant situé au-dessus et au-dessous d'un convoyeur à bande sous aération et transportant des objets à stériliser ou désinfecter.

13. Utilisation d'un appareil selon l'une quelconque des revendications 1 à 10 pour la stérilisation de matériel médical, chirurgical ou dentaire.

14. Utilisation d'un appareil selon la revendication 11 pour la désinfection de locaux et de matériel, de préférence dans le secteur hospitalier et agroalimentaire.

15. Utilisation d'un appareil selon la revendication 11 lors d'interventions chirurgicales humaines ou animales, directement sur le champ opératoire "ouvert".

16. Utilisation d'une installation selon la revendication 12 pour la stérilisation de produits agroalimentaires.
